# EUROPEAN PATENT SPECIFICATION

(11) **EP 2 630 926 B1**
(45) Date of publication and mention of the grant of the patent: **17.02.2016**
(21) Application number: 13156276.1
(22) Date of filing: 22.02.2013
(51) Int. Cl.: A61B 17/34

(54) **Two-part access port**
Zweiteiliger Zugangsanschluss
Orifice d'accès en deux parties

(30) Priority: 23.02.2012 US 201261602102 P; 22.01.2013 US 201313746520
(43) Date of publication of application: 28.08.2013
(73) Proprietor: Covidien LP, Mansfield, MA 02048 (US)
(72) Inventor: Kleyman, Gennady, Brooklyn, NY New York 11230 (US)
(74) Representative: Soames, Candida Jane

(56) References cited:
- EP-A2- 2 308 399
- WO-A2-2006/110733
- US-A1- 2004 073 090
- US-A1- 2005 090 717

## Description

### BACKGROUND

### 1. Technical Field

The present disclosure relates to a surgical apparatus for use during a minimally invasive surgical procedure. More particularly, the present disclosure relates to a surgical access apparatus adapted for insertion into an opening in tissue, and, for the sealed reception of one or more surgical objects such that a substantially fluid-tight seal is formed with both the tissue and the surgical object, or objects.

### 2. Background of the Related Art

Minimally invasive surgery, e.g., laparoscopic, endoscopic, and thoroscopic surgery, has many advantages over traditional open surgeries. In particular, minimally invasive surgery eliminates the need for a large incision, thereby reducing discomfort, recovery time, and many of the deleterious side effects associated with traditional open surgery.

Minimally invasive surgeries are performed through small openings in a patient's skin. These openings may be incisions in the skin or may be naturally occurring body orifices (e.g., mouth, anus, or vagina). During a typical minimally invasive procedure, surgical objects, such as surgical access devices, e.g., trocar and cannula assemblies, or endoscopes, are inserted into the patient's body through the incision in tissue. In general, insufflation fluid (e.g., CO₂ or saline) is used to enlarge the area surrounding the target surgical site to create a larger, more accessible work area. Accordingly, the maintenance of a substantially fluid-tight seal is desirable so as to minimize the escape of the insufflation fluids and the deflation or collapse of the enlarged surgical site. To this end, various valves and seals are used during the course of minimally invasive procedures and are widely known in the art.

Document US20050090717, which forms the basis of the preamble of claim 1, discloses a surgical access device with proximal and distal portions, the said proximal and distal portions being interconnected with a liner, and a detachable holder to allow adjustment of the distance between the proximal and distal portions.

Removal of a specimen from a surgical site is desirable during many minimally invasive procedures. The specimen may be wider than an access opening of a valve or seal, thus requiring a removal of the valve or seal before the specimen can be removed, which may result in increased duration, complexity, and/or cost of the procedure. A continuing need exists for a surgical access apparatus that can be used both for maintaining the integrity of an insufflated workspace and for facilitating a removal of a specimen from the workspace.

### SUMMARY

A surgical access apparatus has a body including a proximal portion, a distal portion, and a liner interconnecting the proximal portion and the distal portion. The proximal portion and the distal portion are longitudinally movable relative to each other. A holder is releasably attached to the body for holding the body in a first state, in which the proximal portion and the distal portion are a first distance apart. Removing the holder from the body allows the body to transition to a second state, in which the proximal portion and the distal portion are a second distance apart. The second distance is greater than the first distance. At least one port extends through the proximal portion and is adapted for the reception of an object. Compressible material defining the at least one port is adapted to deform to establish a substantial sealed relation with the object.

The holder may be a suture. The holder may be welded to the body. The distal portion may have an aperture substantially wider than each of the at least one port. The liner may be collapsed in the first state and expanded in the second state. The distal portion may include a bag portion for carrying a specimen. The compressible material may be a foam or a plastic..

An exemplary method of retrieving a specimen has the steps of inserting a surgical access apparatus into an opening, releasing a holder from the body of the surgical access apparatus, moving a proximal portion and a distal portion of the body longitudinally apart, moving a specimen within the liner of the body, and removing the body from the opening. The liner interconnects the proximal portion and the distal portion. The body further defines at least one port configured for forming a substantial sealed relation with a surgical instrument inserted through the at least one port.

The releasing step may include cutting the holder. The releasing step may include removing the holder from the body. The surgical access apparatus may include a bag for carrying a specimen. The step of moving a proximal portion and a distal portion may include stretching the liner. The step of moving a specimen within the liner may be performed by a surgical instrument inserted through the at least one port.

### BRIEF DESCRIPTION OF THE DRAWINGS

The above and other aspects, features, and advantages of the present disclosure will become more apparent in light of the following detailed description when taken in conjunction with the accompanying drawings in which:
**FIG. 1** is a side, perspective view of a surgical access apparatus according to an embodiment of the present disclosure;
**FIG. 2** is a side, cross-section view of the surgical access apparatus of **FIG. 1** in a first configuration;
**FIG. 3** is a side, cross-section view of the surgical access apparatus of **FIG. 1** with holders removed in a second configuration;
**FIG. 4** is a side, cross-section view of the surgical access apparatus of **FIG. 1** in a first condition prior to insertion thereof within an opening in a tissue;
**FIG. 5** is a side, cross-section view of the surgical access apparatus of **FIG. 1** inserted through an opening in tissue and having a holder cut;
**FIG. 6** is a side, cross-section view of the surgical access apparatus of **FIG. 1** in a second configuration in an opening in tissue and having a surgical instrument inserted therethrough;
**FIG. 7** is a side, cross-section view of the surgical access apparatus of **FIG. 1** having a specimen placed within a liner thereof; and
**FIG. 8** is a side, cross-section view of the surgical access apparatus of **FIG. 1** removed from an opening in a tissue and having a specimen therein.

### DETAILED DESCRIPTION

Particular embodiments of the present disclosure are described hereinbelow with reference to the accompanying drawings; however, it is to be understood that the disclosed embodiments are merely exemplary of the disclosure and may be embodied in various forms. Well-known functions or constructions are not described in detail to avoid obscuring the present disclosure in unnecessary detail. Therefore, specific structural and functional details disclosed herein are not to be interpreted as limiting, but merely as a basis for the claims and as a representative basis for teaching one skilled in the art to variously employ the present disclosure in virtually any appropriately detailed structure.

Like reference numerals may refer to similar or identical elements throughout the description of the figures. As shown in the drawings and described throughout the following description, as is traditional when referring to relative positioning on a surgical instrument, the term "proximal" refers to the end of the apparatus which is closer to the user and the term "distal" refers to the end of the apparatus which is farther away from the user. The term "clinician" refers to any medical professional (i.e., doctor, surgeon, nurse, or the like) performing a medical procedure involving the use of embodiments described herein.

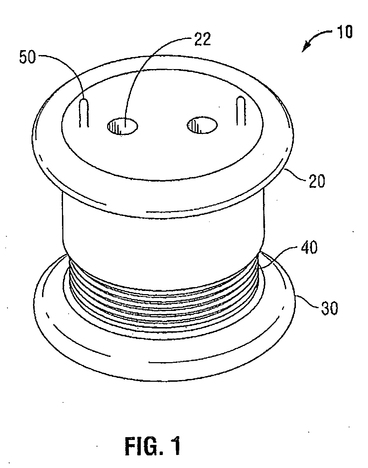

**FIG. 1** illustrates a surgical access apparatus **10** for use in a surgical procedure, e.g., a minimally invasive procedure. Surgical access apparatus **10** has a body including a proximal portion **20**, a distal portion **30**, and a liner **40** interconnecting proximal portion **20** and distal portion **30**. Proximal portion **20** includes at least one port **22** extending longitudinally therethrough. At least one port **22** is configured for removably receiving a surgical object therethrough.

Surgical access apparatus **10** may be composed of any suitable combination of biocompatible materials, such as biocompatible metals, plastics, and foams. In one embodiment, surgical access apparatus **10** includes a foam sufficiently compliant to accommodate off-axis motion of a surgical object. The foam may be a polyisoprene material. Proximal portion **20** may be composed of a material having sufficient compliance to form a substantially fluid-tight seal about one or more surgical objects within at least one port **22**. At least one port **22** has an initial diameter without an object inserted therethrough. The initial diameter may be substantially zero mm. Insertion of a surgical object through at least port **22** expands at least port **22** from the initial diameter to at least a diameter of the surgical object. Resilient material defining at least one port **22** attempts to resiliently restore at least one port **22** to the initial diameter, thereby forming a substantially fluid-tight seal about the surgical object. Proximal portion **20** and distal portion **30** may be composed of a resiliently compressible material to allow surgical access apparatus **10** to resiliently transition between a compressed condition to facilitate insertion of surgical apparatus **10** and an expanded condition to substantially secure surgical access apparatus **10** within a bodily opening. After being compressed from an uncompressed state for insertion, the resiliently compressible material defining proximal portion **20** and distal portion **30** attempts to resiliently expand to the uncompressed state, thereby forming a substantially fluid-tight seal with tissue defining a bodily opening.

Proximal portion **20** may have a proximal end sufficiently wide to inhibit surgical access apparatus **10** from passing completely through a bodily opening during a surgical procedure. Distal portion **30** may likewise have a distal end sufficiently wide to inhibit unintended removal of surgical access apparatus **10** from a bodily opening during a surgical procedure. The proximal end and the distal end may be planar. In another embodiment, the proximal end and the distal end may be substantially arcuate to assist in the insertion of surgical apparatus **10** within a bodily opening. Proximal end **20** may have a body portion distal to the proximal end thereof having a diameter appreciably less than a diameter of the proximal end. Distal portion **30** may likewise have a body portion proximal to the distal end thereof having a diameter appreciably less than a diameter of the distal end. Surgical apparatus **10** may define an "hour-glass" shape or configuration to assist in anchoring surgical apparatus **10** within tissue. In alternate embodiments, proximal end **20** and distal end **30** may have a substantially uniform diameter. The body portions of proximal portion **20** and distal portion **30** may exhibit any suitable configuration, **e.g**., substantially circular, oval, or oblong.

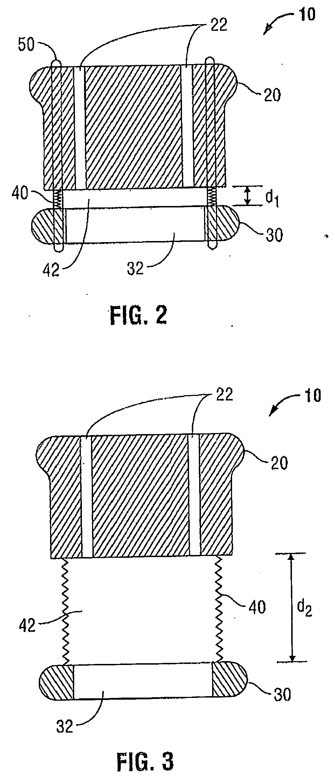

As seen in **FIG. 2**, surgical access apparatus **10** includes a holder **50** for holding the body of surgical access apparatus **10** in a first state. In the first state, proximal portion **20** and distal portion **30** are a first distance **d₁** longitudinally apart. The first distance **d₁** is preferably minimal. Proximal portion **20** and distal potion **30** may be in contact. Liner **40** is compressed, folded, crumpled, or otherwise shortened along a longitudinal dimension of surgical access apparatus **10** to assist in minimizing the first distance **d₁.**

Holder **50** inhibits proximal portion **20** and distal portion **30** from moving longitudinally apart beyond the first distance **d₁.** Surgical access apparatus **10** may include more than one holder **50**. Holder **50** extends from a proximal end of proximal portion **20** through the body to distal portion **30**. In one embodiment, holder **50** is a suture. In another embodiment, holder **50** is welded to the body. A portion of holder **50** on the proximal end of proximal portion **20** may be cut, pulled, disengaged, or otherwise released from the body to allow the body to transition to a second state.

Distal portion **30** has an aperture **32** defined therein. Aperture **32** is sufficiently wide for a specimen to pass therethrough. Aperture **32** also allows passage of surgical instruments therethrough. Aperture **32** may be wider than each of at least one port **22**.

Liner **40** is sufficiently longitudinally expandable for a space **42** therein to be sufficiently large to receive a specimen therein. Liner **40** may longitudinally extend through an entire depth of an opening in a fully expanded state. Space **42** may be wider than aperture **32**, thus allowing distal portion **30** to assist in carrying a specimen within space **42**. In some embodiments, liner **40** may include a net, bag, or other carrying device for carrying a specimen therein. Liner **40** may be composed of a relatively thin, flexible material adapted for compression and expansion. Liner **40** is substantially impermeable to reduce seeding or spreading of the specimen.

As seen in **FIG. 3** the body of surgical access apparatus **10** is in a second state. Holder **50** is removed from the body of surgical access apparatus **10** and no longer inhibits longitudinal movement of proximal portion **20** relative to distal portion **30**. Liner **40** is longitudinally expanded, thereby increasing a volume of space **42**. Proximal end **20** and distal end **30** are at a second distance **d₂** from each other greater than the first distance **d₁**.

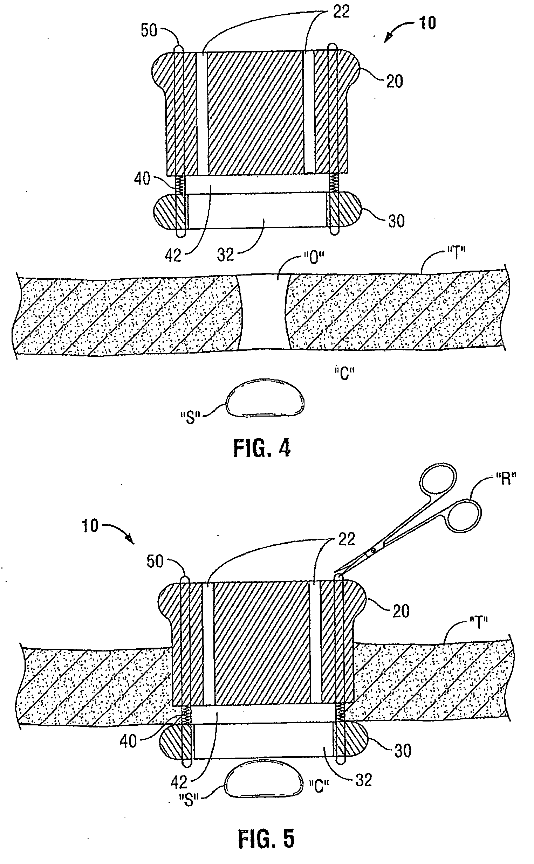

Turning to **FIGS. 4-8**, an exemplary method of use of surgical access apparatus **10** is illustrated. As seen in **FIG. 4**, an opening "**O**" exists in a tissue "**T**" through which a cavity "C" may be accessed. A specimen "**S**" exists in cavity "**C**". Surgical access apparatus **10** is compressible to a diameter less than a diameter of opening "**O**" to facilitate insertion of surgical access apparatus **10** in opening "**O**". The body of surgical access apparatus **10** is in the first state when inserted. When surgical access apparatus **10** is within opening "**O**", surgical access apparatus **10** resiliently expands until it forms a substantial sealed relation with tissue "**T**". Surgical instruments may then be inserted through at least one port **22** and continue through space **42** and aperture **32** to access cavity "**C**".

As seen in **FIG. 5**, once specimen "**S**" is ready for removal, holder **50** may be cut by a cutting instrument "**R**" to release holder **50** from the body of surgical access apparatus **10**. Holder **50** may then be pulled proximally for removal thereof from the body.

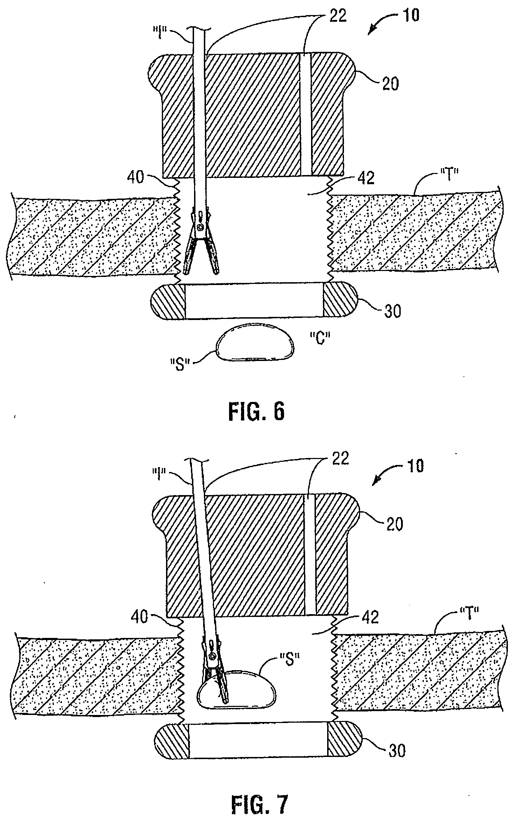

As seen in **FIG. 6**, the body of surgical access apparatus **10** is transitioned to the second state. Distal portion **30** is positioned distal to tissue "**T**". Proximal portion **20** is pulled proximally until it is positioned fully proximal to tissue "**T**". Liner **40** expands longitudinally along an entire depth of opening "**O**". A grasping instrument "**I**" is then inserted through at least one port **22**. Compressible material defining at least one port **22** forms a substantially fluid-tight seal around grasping instrument "**I**".

As seen in **FIG. 7**, grasping instrument "**I**" grasps specimen "**S**" and pulls specimen "**S**" through aperture **32** into space **42**. Grasping instrument "**I**" may continue to grasp specimen "**S**" during removal. In embodiments having a carrying device such as a bag, specimen "**S**" may be placed within the carrying device by grasping instrument "**I**". Alternatively, a carrying device could be provided by a surgical instrument inserted through at least one port **22**.

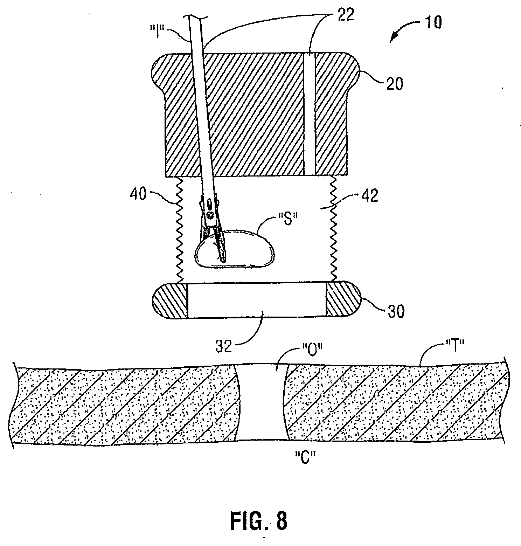

As seen in **FIG. 8**, surgical access apparatus **10** is removed from opening "**O**" to complete the exemplary removal method. Distal portion **30** is compressed to a diameter less than a diameter of opening "**O**". Surgical access apparatus **10** may then be pulled proximally from tissue "**T**". If no carrying device is provided, grasping instrument "**I**" is pulled proximally with surgical access apparatus **10** to keep specimen "**S**" within space **42** as surgical access apparatus **10** is removed from opening "**O**". Distal portion **30** resiliently expands upon removal from opening "**O**".

It should be understood that the foregoing description is only illustrative of the present disclosure. Various alternatives and modifications can be devised by those skilled in the art without departing from the disclosure. Accordingly, the present disclosure is intended to embrace all such alternatives, modifications and variances. The embodiments described with reference to the attached drawing figs. are presented only to demonstrate certain examples of the disclosure. Other elements that are insubstantially different from those described above and/or in the appended claims are also intended to be within the scope of the disclosure.

## Claims

1. A surgical access apparatus (10) comprising:
a body including a proximal portion (20), a distal portion (30), and a liner (40) interconnecting the proximal portion and the distal portion, the proximal portion and the distal portion being longitudinally movable relative to each other;
at least one port (22) extendin through the proximal portion and being adapted for the reception of an object whereby compressible material defining the at least one port is adapted to deform to establish a substantial sealed relation with the object;
a holder (50) releasably attached to the body for holding the body in a first state, wherein the proximal portion and the distal portion are a first distance apart,
**characterised in that** the holder extends from the proximal end of the proximal portion to the distal portion and releasing the holder from the body allows the body to transition to a second state, wherein the proximal portion and the distal portion are a second distance apart, the second distance being greater than the first distance.

2. The surgical access apparatus of claim 1, wherein the holder is a suture.

3. The surgical access apparatus of claim 1 or claim 2, wherein the holder is welded to the body.

4. The surgical access apparatus of any preceding claim, wherein the distal portion has an aperture (32) substantially wider than each of the at least one port.

5. The surgical access apparatus of any preceding claim, wherein the liner is collapsed in the first state and expanded in the second state.

6. The surgical access apparatus of any preceding claim, wherein the distal portion includes a carrying device for carrying a specimen therein.

7. The surgical access apparatus of any preceding claim, wherein the compressible material is a foam.

8. The surgical access apparatus of any of claims 1 to 6, wherein the compressible material is a plastic.

## Patentansprüche

1. Chirurgische Zugangsvoruchtung (10), die umfasst:
einen Körper, der einen proximalen Abschnitt (20), einen distalen Abschnitt (30) und ein Tragband (40) enthält, das den proximalen Abschnitt und den distalen Abschnitt miteinander verbindet, wobei der proximale Abschnitt und der distale Abschnitt in Bezug auf einander längswärts beweglich sind;
zumindest eine Öffnung (22), die sich durch den proximalen Abschnitt erstreckt und so ausgelegt ist, dass sie ein Objekt aufnimmt, wobei komprimierbares Material, das die zumindest eine Öffnung definiert, so ausgelegt ist, dass es sich verformt, um ein im Wesentlichen abgedichtetes Verhältnis zum Objekt zu erreichen;
eine Halterung (50), die lösbar am Körper angebracht ist, um den Körper in einem ersten Zustand zu halten, in dem der proximale Abstand und der distale Abstand um einen ersten Abstand beabstandet sind,
**dadurch gekennzeichnet, dass** sich die Halterung vom proximalen Ende des proximalen Abschnitts zum distalen Ende erstreckt und das Lösen der Halterung vom Körper ermöglicht, dass der Körper in einen zweiten Zustand übergeht, in dem der proximale Abstand und der distale Abstand um einen zweiten Abstand beabstandet sind, wobei der zweite Abstand größer als der erste Abstand ist.

2. Chirurgische Zugangsvorrichtung nach Anspruch 1, wobei die Halterung eine Naht ist.

3. Chirurgische Zugangsvoruchtung nach Anspruch 1 oder 2, wobei die Halterung mit dem Körper verschweißt ist.

4. Chirurgische Zugangsvorrichtung nach einem vorstehenden Anspruch, wobei der distale Abschnitt zumindest ein Loch (32) aufweist, das im Wesentlichen breiter als jede der zumindest einen Öffnung ist.

5. Chirurgische Zugangsvoruchtung nach einem vorstehenden Anspruch, wobei das Trägerband im ersten Zustand gefaltet und im zweiten Zustand ausgedehnt ist.

6. Chirurgische Zugangsvoruchtung nach einem vorstehenden Anspruch, wobei der distale Abschnitt eine Trägereinrichtung zum Tragen einer Probe darin enthält.

7. Chirurgische Zugangsvorrichtung nach einem vorstehenden Anspruch, wobei das komprimierbare Material ein Schaumstoff ist.

8. Chirurgische Zugangsvorrichtung nach einem der Ansprüche 1 bis 6, wobei das komprimierbare Material ein Kunststoff ist.

## Revendications

1. Appareil d'accès chirurgical (10) comprenant :
un corps incluant une partie proximale (20), une partie distale (30) et une doublure (40) reliant la partie proximale et la partie distale, la partie proximale et la partie distale étant mobiles de façon longitudinale l'une par rapport à l'autre ;
au moins un orifice (22) s'étendant à travers la partie proximale et étant conçu pour la réception d'un objet de sorte qu'une matière compressible définissant l' au moins un orifice est conçue pour se déformer pour établir une relation scellée substantielle avec l'objet ;
un support (50) attaché de façon amovible au corps pour maintenir le corps dans un premier état, dans lequel la partie proximale et la partie distale sont espacées d'une première distance,
**caractérisé en ce que** le support s'étend depuis l'extrémité proximale de la partie proximale jusqu'à la partie distale et la libération du support depuis le corps permet au corps d'effectuer une transition vers un second état, dans lequel la partie proximale et la partie distale sont espacées d'une seconde distance, la seconde distance étant plus grande que la première distance.

2. Appareil d'accès chirurgical selon la revendication 1, dans lequel le support est une suture.

3. Appareil d'accès chirurgical selon la revendication 1 ou la revendication 2, dans lequel le support est soudé au corps.

4. Appareil d'accès chirurgical selon n'importe quelle revendication précédente, dans lequel la partie distale a une ouverture (32) sensiblement plus large que chacun de l'au moins un orifice.

5. Appareil d'accès chirurgical selon n'importe quelle revendication précédente, dans lequel la doublure est pliée dans le premier état et étendue dans le second état.

6. Appareil d'accès chirurgical selon n'importe quelle revendication précédente, dans lequel la partie distale inclut un dispositif de transport pour transporter un spécimen en son sein.

7. Appareil d'accès chirurgical selon n'importe quelle revendication précédente, dans lequel la matière compressible est une mousse.

8. Appareil d'accès chirurgical selon n'importe laquelle des revendications 1 à 6, dans lequel la matière compressible est un plastique.
